Europäisches Patentamt

(19) European Patent Office

Office européen des brevets

(11) **EP 0 427 741 B1**

(12) **EUROPEAN PATENT SPECIFICATION**

(45) Date of publication and mention
of the grant of the patent:
**07.01.1998 Bulletin 1998/02**

(21) Application number: **89907896.8**

(22) Date of filing: **13.06.1989**

(51) Int. Cl.⁶: **A61L 15/16**, A61K 9/70

(86) International application number:
**PCT/US89/02561**

(87) International publication number:
**WO 89/12470 (28.12.1989 Gazette 1989/30)**

(54) **SUBSATURATED TRANSDERMAL DELIVERY DEVICE**

UNTERGESÄTTIGTE TRANSDERMALE ABGABEVORRICHTUNG

DISPOSITIF DE DISTRIBUTION TRANSCUTANEE SOUS-SATUREE

(84) Designated Contracting States:
**AT BE CH DE FR GB IT LI LU NL SE**

(30) Priority: **14.06.1988 US 206546**
**14.12.1988 US 284283**

(43) Date of publication of application:
**22.05.1991 Bulletin 1991/21**

(73) Proprietor: **ALZA CORPORATION**
**Palo Alto California 94303-0802 (US)**

(72) Inventors:
 • **OSBORNE, James L.**
 **Mountain View, CA 94043 (US)**
 • **NELSON, Melinda**
 **Sunnyvale, CA 94087 (US)**
 • **ENSCORE, David James**
 **Saratoga, CA 95070 (US)**
 • **YUM, Su Il**
 **Los Altos, CA 94022 (US)**
 • **GALE, Robert M.**
 **Los Altos, CA 94022 (US)**
 • **CAMPBELL, Patricia S.**
 **Palo Alto, CA 94301 (US)**

(74) Representative:
**Evans, David Charles et al**
**F.J. CLEVELAND & COMPANY**
**40-43, Chancery Lane**
**London, WC2A 1JQ (GB)**

(56) References cited:
**EP-A- 0 305 757        DE-A- 3 523 065**
**US-A- 4 379 454**

**Description**

This invention relates to transdermal delivery devices intended to deliver biologically active agents through skin at substantially constant rates for extended periods of time and more particularly to such devices in which the active agent to be delivered is present in the device at a concentration below saturation.

BACKGROUND OF THE INVENTION

Transdermal delivery devices for the delivery of a wide variety of biologically active agents have been known for sometime and representative systems are disclosed in U.S. Patents 3,598,122, 3,598,123, 3,742,951, 4,031,894, 4,060,084, 4,144,317, 4,201,211 and 4,379,454 which are incorporated herein by reference. Such devices generally comprise an impermeable backing, a drug or active agent reservoir, a rate controlling membrane and a contact adhesive layer which can be laminated or heat sealed together to produce a transdermal delivery device. Although subsaturated systems are known, see patent 4,379,454, for example, it is generally desirable that the agent reservoir comprise the agent to be delivered in a suitable carrier at a concentration above the saturation concentration in the carrier. This is done to maintain a unit activity source of the agent so that the delivery rate of the agent will remain substantially constant over the intended administration period; the amount of agent originally present over saturation being the depot or reservoir for the dose of agent ultimately delivered. If the concentration of the agent drops below unit activity during the delivery period, the rate of agent delivery will also tend to decrease. It is also generally desirable to minimize the residual agent in the device after use and, to accomplish this, devices normally utilize a carrier, which has a limited solubility for the agent to be delivered. Although such typical devices have been found useful for the delivery of a wide variety of agents, we have encountered significant problems in producing devices intended to deliver an agent which is capable of dissolving or plasticizing medically acceptable contact adhesives. Such agents are usually, but not always, oily, nonpolar materials, liquid at ambient temperatures, and are either solvents for medically acceptable contact adhesives or are highly soluble therein and cause such adhesives to loose their adhesiveness. In the latter case, the agent, may not actually solvate the adhesive but instead plasticize the adhesive and cause it to swell, loose its cohesiveness and adhesiveness, and degrade its other physical properties. As used herein, an agent is a "solvent" for medically acceptable adhesives, and such adhesives are "soluble" in such agents if the agent either dissolves or plasticizes such adhesives as described above.

Agents which are such solvents may be drugs, permeation enhancers or other transdermally deliverable substances. Representatives of such agents are drugs such as benztropine base, an anticholinergic useful in the treatment of Parkinsonism, the antispasmolytic drugs secoverine and dexsecoverine, nicotine, useful in the withdrawal from smoking, and arecoline, a cholinergic and anthelmintic agent. Representative permeation enhancers include polyethylene glycol monolaurate (PGML), glycerol monolaurate (GML), and glycerol monooleate (GMO) and ethanol. Although ethanol is not an oily, nonpolar liquid, it is an example of a material which, in high concentrations, can act as solvent for certain medically acceptable contact adhesives.

Regardless of the initial concentration of the agent in the reservoir and adhesive layers, the devices will equilibrate upon standing. Thus, if the agent is a solvent for the adhesive layer, we have found that substantial quantities migrate from the reservoir through the rate controlling membrane and into the adhesive layer prior to use. The migration will continue until the thermodynamic activity of the agent in the adhesive equals the activity of the agent in the reservoir. Thus, a substantial amount of agent can migrate into the adhesive layer and will be released onto the skin in an uncontrolled manner before the rate controlling membrane can exert any effect on the agent remaining in the reservoir. Also, high concentrations of agent in the adhesive layer and in direct contact with the skin may cause irritation or produce undesirably high plasma levels during the initial period after application to the skin and prior to depletion of the initial loading of agent in the contact adhesive layer. In addition to the deleterious effects on a patient that may be caused by high concentrations of agent in the adhesive, certain adhesives tend to lose their adhesive properties when they are dissolved or plasticized by the agent being delivered.

According to one aspect of the present invention there is provided a transdermal delivery device for delivering an agent over a predetermined administration period comprising a reservoir containing an agent and a diluent, release rate controlling means through which said agent, but not said diluent, permeates in use from the device to the skin of a patient, and in-line adhesive means through which said agent must pass to reach the skin, wherein said agent is a solvent for the adhesive, characterised in that the initial equilibrated activity of said agent in said reservoir is below saturation and at a level at which the adhesive means retains adhesive properties, and the initial loading of the agent in the reservoir is sufficient to prevent the activity of the agent in the reservoir from decreasing by more than 75% during the predetermined administration period.

According to a different aspect of the invention there is provided a method for administering nicotine which comprises:

(a) applying a device in accordance with the invention to the skin of a patient when the thermodynamic activity of said nicotine in said reservoir is no greater than 0.50; and

(b) maintaining the concentration of nicotine in said reservoir throughout a predetermined administration period at a level sufficient to prevent the activity from decreasing by more than 75%.

The invention, in yet another aspect, also provides a method for administering nicotine which comprises:

(a) applying a device in accordance with the invention to the skin of the patient; and

(b) removing it at the end of said predetermined administration period.

According to our invention we have provided a rate controlled, subsaturated transdermal delivery device having an in-line adhesive which delivers an agent which is a solvent for the in-line adhesive and which exhibits improved release characteristics. In certain embodiments of our invention a substantially constant release rate over a substantial portion of a predetermined administration period can be obtained. The subsaturated reservoir may contain a sufficient amount of agent to prevent the activity from decreasing by more than about 75%, and preferably no more than about 25%, during a predetermined delivery period of up to 7 days. The device is also preferably designed such that no more than, and preferably substantially less than, half of the total agent loading in the device is in the adhesive and rate controlling membrane layers after equilibration and prior to use.

Preferred embodiments of our invention are rate-controlled drug delivery devices having in-line adhesives for the controlled delivery of drugs which are solvent for the in-line adhesive such as the smoke cessation aid, nicotine, the anticholinergic, benztropine, and the tertiary amine secoverine, 1-cyclohexyl-4-C[ethyl(p-methoxy-alpha-methylphenylethyl) amino]-butazone, an anti-spasmodic agent described in U.S. Patents 3,996,245 and 4,125,623 which are incorporated herein by reference. The active, (d) isomer of secoverine is hereinafter referred to as "dexsecoverine".

Other preferred embodiments can be used to deliver drugs in connection with permeation enhancers such as ethanol, PGML, GML and GMO for example. Attempts to produce transdermal delivery devices for these agents and enhancers by following the aforementioned teachings of the prior art were unsuccessful based on a combination of the above considerations. It is also expected that similar problems will be encountered with respect to other agents which are solvents for medical adhesives and this invention will have utility with such other agents.

It is accordingly an object of this invention to provide a rate controlled transdermal delivery device having an in-line adhesive and a subsaturated agent reservoir which device exhibits improved agent release rate characteristics.

It is another object of this invention to provide a transdermal delivery device for the delivery of agents which are solvents (as defined herein) for in-line adhesives.

It is another object of this invention to improve the delivery characteristics of a rate-controlled, transdermal delivery device utilizing a subsaturated agent reservoir.

These and other objects of the invention will be readily apparent from the following description with reference to the accompanying drawings wherein:

Figure 1 is a cross section through an embodiment of the transdermal delivery devices according to this invention;
Figure 2 is a cross section through another embodiment of a transdermal delivery device according to this invention;
Figures 3, 5 ,6 and 7 are plots of *in vitro* release rates directly into a sink at 32#C (Fig. 3) or 35#C (Figs 5,6 & 7) vs. time for embodiments of this invention; and
Figure 4 compares plots of its *in vitro* release rates at 32#C directly into a sink vs. time with the *in vitro* flux at 32#C through human cadaver skin into a sink vs. time obtained from an embodiment of this invention.

DESCRIPTION OF THE INVENTION

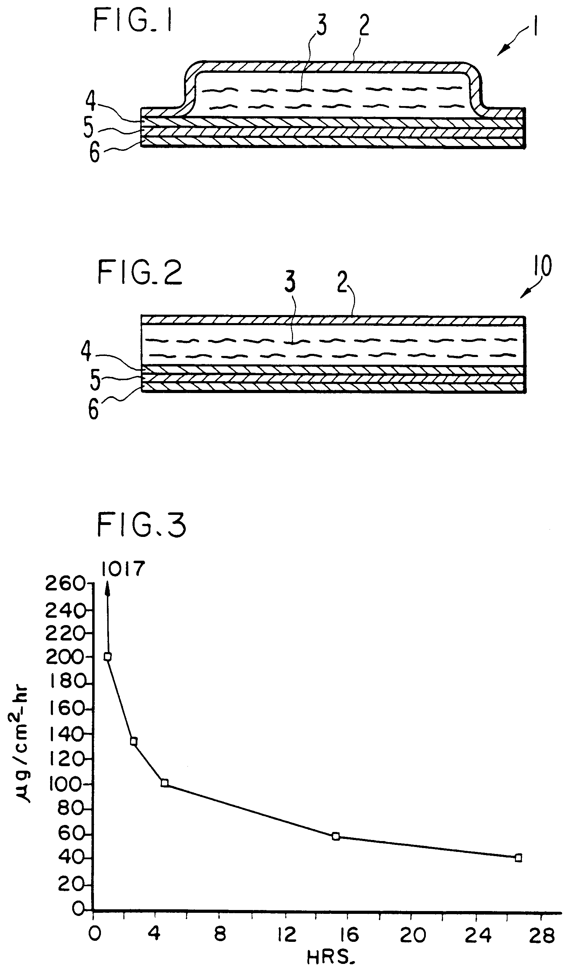

Referring now to Figures 1 and 2 (like reference numerals referring to common elements), transdermal delivery devices 1 and 10 according to this invention are shown. Devices 1 and 10 are formed of an impermeable backing 2, an agent reservoir 3, an agent release rate controlling membrane 4, a contact adhesive 5 permeable to the agent, and a release liner 6 adapted to be removed from the adhesive layer prior to application to the skin of the subject to whom the agent is to be administered. As noted above, the agent to be delivered is a solvent for the adhesive forming the adhesive layer 5. In this regard, the reservoir may contain more than one agent according to this invention provided that at least one of the agents is a solvent for the adhesive. Typically, one of the agents could be a drug and another agent could be a permeation enhancer or another drug, for example.

The embodiments of Figures 1 and 2 differ in that the agent reservoir 3 of the embodiment of Figure 1 is less viscous than the reservoir 3 of Figure 2 such that the impermeable backing 2 is bonded at its periphery to the rate controlling membrane 4 to form a pouch fully enclosing reservoir 3 to prevent it from flowing or oozing. In the embodiment of Figure 2 the reservoir 3 has sufficient viscosity to maintain its structural integrity without a peripheral or circumferential seal. Although Figures 1 and 2 relate to laminated devices, other arrangements of the adhesive, reservoir and rate controlling membranes are usable and include, for example, an adhesive having microcapsules of the agent within a rate controlling membrane dispersed therethrough as shown in aforementioned patent No. 3,598,123.

According to this invention, transdermal delivery devices 1 and 10 are intended to be applied to a patient for a predetermined administration period, typically from about 1-7 days. The predetermined administration period may be in the range 8 hours to 3 days. During the administration period it would be desirable to control the amount of agent that is released from the device so that the agent can be administered to the patient in a predetermined and controlled manner. The in vitro agent release rate or flux from a transdermal delivery device directly into an infinite sink as a function of time can be considered to consist of two phases, a first, initial "transient" phase, and a second, subsequent "steady-state" delivery phase. During the initial transient phase, the agent is released at a high rate as a result of the initial loading of the agent in the adhesive and rate controlling membrane layers 5 and 4, respectively. This initial pulse release decreases relatively rapidly as a function of $t^{-1/2}$ until the initial loading of agent in the adhesive layer is depleted and the "steady-state" phase in which agent is being delivered from reservoir 3 commences.

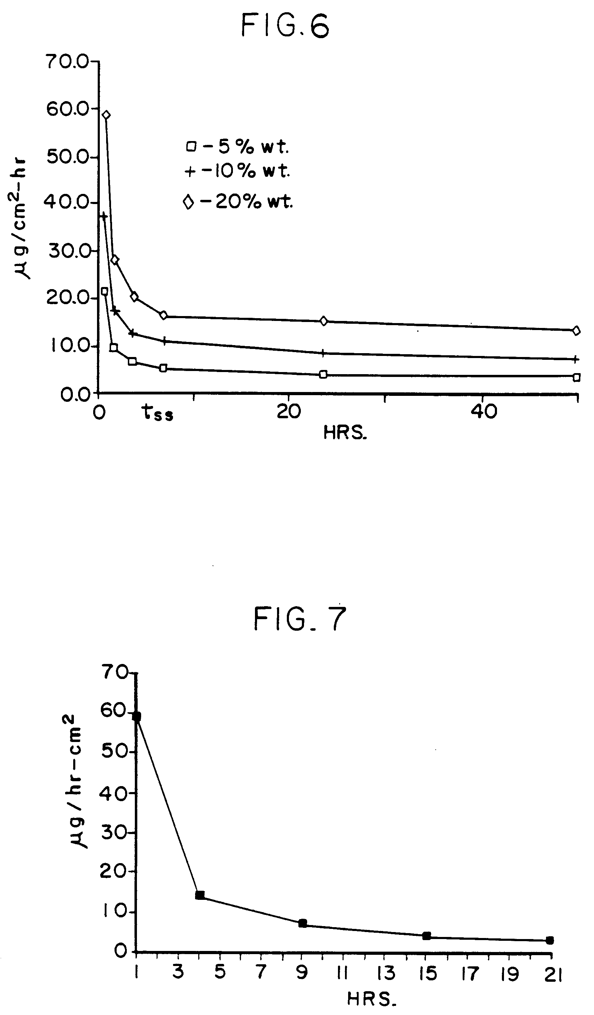

$t_{ss}$ shown in Figure 5 and 6 represents the time at which the initial transient phase ends and the steady state delivery phase commences. The variation of release rate with time during the steady-state phase depends on the structure of the device. Simple monoliths of the prior art exhibit a theoretical variation of release rate as a function of $t^{-1/2}$, whereas prior art devices having unit activity reservoirs and release rate-controlling membranes exhibit theoretical release rates that vary with $t^0$, i.e., they remain constant. Devices according to this invention exhibit a theoretical release rate which varies as a function of $t^n$ where $-\frac{1}{2} \leqq n < 0$ and preferred embodiments exhibit in vitro release rates which approach those obtained from zero order devices.

According to preferred embodiments of this invention, the steady-state in vitro release rate can be maintained substantially constant from the termination of the initial transient phase until the expiration of the predetermined administration period. As used herein, the in vitro agent delivery rate is considered to be "substantially constant" if the steady-state rate does not vary more than about ±50%, and preferably no more than ±25%, during the steady state administration period.

As used herein, the term "agent" is used in its broadest sense to mean any material which is to be delivered into the body of a human or animal to produce a beneficial, therapeutic or other intended effect, such as permeation enhancement, for example, and is not limited to drugs and pharmaceutical products. The maximum allowable concentration of the agent in the adhesive will be determined by such factors as the agent concentration at which the adhesive properties are impaired, the agent concentration at which irritation problems or unacceptably high initial transdermal agent fluxes, for example, are observed. When such undesirable effects occur, it is necessary that the initial activity of the agent in the adhesive be at a lower level. Because the device will equilibrate on standing, the activity (but not necessarily the concentration) of the agent in the adhesive will ultimately be the same as the activity of the agent in the reservoir layer.

Transdermal delivery devices, according to our invention, have the following characteristics:

1. The devices utilize an in-line adhesive to maintain the device on the skin;
2. The agent to be delivered is a solvent for the in-line adhesive;
3. The initial equilibrated concentration of the agent in the reservoir 3 is below saturation; the equilibrated activity being less than 0.50;
4. The reservoir 3 comprises the agent dissolved in a diluent with respect to which rate controlling membrane 4 is substantially impermeable;
5. The initial loading of the agent in reservoir 3 may be sufficient to prevent the activity of the agent in the reservoir from decreasing by more than about 75% and preferably no more than about 25% during a predetermined period of administration of 7 days; and
6. The thickness of the adhesive, rate controlling membrane and reservoir layers may be selected so that at least 50% and, preferably at least 75% of the initial equilibrated agent loading is in the reservoir layer.

To design a system according to our invention, the permeability of skin to the agent to be delivered, the amount of agent required to saturate the agent binding sites in the skin, the maximum activity of agent in the adhesive layer that can be tolerated without loss of adhesive properties and without producing undesirable initial drug pulses, skin irritation or undesirable sensations would be determined by suitable in vitro and in vivo tests. Having determined the maximum allowable activity of agent in the adhesive; a somewhat lower initial activity would typically be employed to provide for a factor of safety. In some instances, such as in the initial administration of the agent or where intermittent, as opposed to continuous, delivery periods are prescribed, the initial loading of agent in the adhesive layer 5 and rate controlling membrane 4 may correspond approximately to the amount of agent needed to saturate the agent binding sites in the skin below the delivery device.

In preferred embodiments the equilibrated agent loading in the reservoir layer 3 is selected to be sufficient to enable the total dose of agent delivered during the predetermined administration period to be delivered while maintaining the decrease in activity of the agent in the non-permeating solvent forming reservoir 3 within the limits noted above. The total loading of agent in each layer of the device can be readily varied without changing the activity simply by increasing or decreasing the thickness of the adhesive layer 5 and/or reservoir layer 3, and also by appropriate selection of the total surface area of the device through which agent is delivered. Because the rate controlling membrane can only act as a release rate limiting element on agent which is in the reservoir; the reservoir thickness should be selected, with respect to the thicknesses of the rate controlling membrane and the adhesive layers, such that at least half, and preferably substantially more, of the initial equilibrated agent loading is in the reservoir.

The rate-controlling membrane 4 would be selected such that the flux of the agent through the membrane into an infinite sink is preferably no greater than the *in vitro* flux of the agent through skin (which would produce about 50% device control) and preferably substantially less. If the skin flux is greater than the membrane flux by a factor of about 2.4, for example, approximately 70% of the rate control is obtained from the device. Suitable materials from which the various layers of the device according to this invention can be made are known to the art and many are described in the aforementioned U.S. patents.

Having thus generally described our invention, the following description and examples will illustrate how variations of the above described parameters affect the administration of the agent.

Device according to our invention can be used for the transdermal administration of nicotine to skin or mucosa. The following calculations can be used to estimate the characteristics required for such a transdermal nicotine delivery device.

Studies with nicotine releasing gum (Nicorette\), have determined that the target blood level of nicotine for reducing the urge to smoke is approximately 12-15 nanograms/ml and that the clearance of nicotine from the body occurs at about 18 ml/min-kg.

In order to deliver adequate amounts of nicotine from a reasonably sized system, the target steady-state *in vivo* delivery rates are within the range of 250-4000 lg/hr with a typical rate being about 1000 lg/hr. This range can be readily achieved according to our invention in a rate controlled device having a size in the range of about 5-50 cm$^2$ and typically about 15-20 cm$^2$. A one day delivery period can readily be obtained from subsaturated devices of this invention, and administration periods of at least 8-10 hours and up to about 3 days can be attained by varying the thickness of the reservoir.

An alternate embodiment of this invention would be a system capable of providing nicotine delivery for 16 hours to be applied each day upon waking, worn all day, and removed and discarded just prior to sleep. This would be repeated for as long as nicotine delivery is desired.

Total nicotine loading in a transdermal delivery device of this invention is preferably at least about 50 mg with the equilibrated concentration of nicotine in the reservoir composition being within the range of 5-50 wt%, corresponding to an activity within the range of 0.05-0.50. Reaction of the skin to nicotine is flux dependent and to minimize skin reaction and it is preferred to maintain the flux below about 200 lg/cm$^2$-hr and preferably below 120 lg/cm$^2$-hr in the steady state phase. Typically the flux will be in the range of about 30 to 70 lg/cm$^2$-hr.

The equilibrated nicotine loading in the reservoir layer is preferably selected to be sufficient to enable the total dose of nicotine delivered during the predetermined administration period to be delivered while maintaining the decrease in activity of the nicotine in the reservoir the limits noted above. The total loading of nicotine in each layer of the device can be readily varied without changing the activity, simply by increasing or decreasing the thickness of the adhesive layer and/or reservoir layer and also by appropriate selection of the total surface area of the device through which nicotine is delivered. Because the rate controlling membrane can only act as a release rate limiting element on the nicotine which is in the reservoir, the reservoir thickness should be selected with respect to the thicknesses of the rate controlling membrane and the adhesive layers, such that at least half, and preferably substantially more, of the initial equilibrated nicotine loading is in the reservoir.

The preferred embodiments of this invention utilize an anhydrous reservoir formed of natural or synthetic rubbers or polymers as known to the art. Ethylene/vinyl acetate copolymer (EVA) may be selected; the VA content may be in the range of about 28-60% by wt. In one embodiment, said reservoir may comprise from 5 to 50 wt % nicotine and from 50 to 95 wt % ethylene vinyl acetate copolymer having a vinyl acetate content in the range 28 to 60 wt %.

The rate controlling membrane may be of a dense polymer film that has the requisite permeability to nicotine. The membrane material would be selected such that the flux of the nicotine through the membrane into a sink is preferably no greater than the in *in vitro* flux of nicotine across skin (which would produce about 50% system control) and preferably substantially less. The fractional control of nicotine delivered across skin (x) from the rate controlled transdermal therapeutic system of this invention is given by the following relationship:

$$x = J_{net}/J_{system}$$

which can be determined from the following equation:

$$Jnet'/Jsystem = [ Jsystem'/Jskin] + 1 ]^{-1}$$

Thus, if the skin flux is greater than the membrane or system flux by a factor of about 2.4, for example, the fractional control of nicotine flux from the system would be:

$$Jnet'/Jsystem = [ (1/2.4) + 1 ]^{-1} = 0.7$$

Therefore, approximately 70% of the rate control is obtained from the system. The flux of nicotine through skin varies somewhat from individual to individual and from body site to body site but generally appears to be in the range of about 400-800 $lg/cm^2/hr$.

Preferably the rate controlling membrane is substantially impermeable to the diluent in which the nicotine in the reservoir is dissolved, although a low permeability to the diluent may not abversely affect the operation of the device. Examples of the types of polymer films that may be used to make the membrane 16 are disclosed in U.S. Pat. Nos. 3,797,494 and 4,031,894, both of which are incorporated herein by reference. Particularly suitable materials for use with the mixture are (EVA), low density polyethylene (LDPE) and high density polyethylene (HDPE).

The composition and thickness of the adhesive layer is selected so as not to constitute a significant permeation barrier to the passage of nicotine. The adhesive material is selected from known materials having a high permeability to nicotine which is also such that it is compatible with nicotine at the activity chosen for the system. Amine resistant silicone adhesives are particularly suitable. These compounds may be modified with silicone oil to obtain the desired tack.

EXAMPLE 1

Transdermal delivery devices for the controlled delivery of nicotine were prepared utilizing a highly permeable, amine resistant adhesive available from Dow Corning (X7-2920), LDPE as the rate controlling membrane, EVA (40% VA) as the non-diffusible drug reservoir diluent, pigmented medium density polyethylene/aluminized polyester as the impermeable backing member and nicotine base as the source of nicotine. The devices had 4 mil LDPE rate controlling membranes, 6 mil drug reservoirs containing either 20 or 25 weight percent nicotine base and a 2 mil adhesive layer. The *in vitro* fluxes of drug from these subsaturated transdermal nicotine devices through cadaver skin into aqueous sink at 35#C were determined and are shown in Table I. Nicotine flux data across skin was obtained from averaging the data generated by devices tested on two different skin donors.

TABLE I

| Time (hr) | Drug Flux with 20 wt% drug ($lg/cm^2$-hr) | Drug Flux with 25 wt% drug ($lg/cm^2$-hr) |
|---|---|---|
| 2 | 87.9 | 133.2 |
| 4 | 65.8 | 104.6 |
| 6 $_{tss}$ | 52.6 | 85.0 |
| 8 | 47.5 | 73.2 |
| 23.25 | 33.4 | 52.8 |
| 27.25 | 27.9 | 45.2 |
| 30.75 | 23.1 | 40.3 |

EXAMPLE II

Subsaturated nicotine transdermal delivery devices (1 $cm^2$) were fabricated having a nicotine loading of about 5 $lg/cm^2$ comprising a 30 wt% nicotine/70 wt% EVA 40 reservoir composition (0.30 nicotine activity), a 2 mil rate controlling membrane and a 2 mil amine resistant adhesive layer (Dow Corning X7-2920 with 5 wt% silicone fluid). The *in vitro* release rate at 35#C directly into an aqueous sink is shown in Figure 3. A device according to this example having a surface area of about 20 $cm^2$ applied to human subjects on a daily basis, should provide transdermal delivery of nicotine at administration rates sufficient to assist in the cessation of smoking.

The previous examples related to nicotine delivery devices; the following examples illustrate embodiments of this invention for transdermally administering other agents.

Secoverine normally exists as a racemic mixture of d and l-isomers, the d-isomer, dexsecoverine, being the biologically active ingredient. We have determined that dexsecoverine diffuses through normal skin at substantially the same rate as the racemic mixture and therefore, if dexsecoverine is used as the agent in the reservoir, the agent flux through the skin need be only about one half that which would otherwise be required if racemic secoverine were delivered.

EXAMPLE III

Transdermal delivery devices for the controlled delivery of dexsecoverine were prepared utilizing Dow Corning DC 355 silicone adhesive as the highly permeable medical adhesive, EVA (9% VA) as the rate controlling membrane, EVA (40% VA) as the non-diffusible drug reservoir diluent, pigmented medium density polyethylene/aluminized polyester as the impermeable backing member and racemic secoverine or dexsecoverine as the source of dexsecoverine. Secoverine and dexsecoverine are extremely soluble (essentially miscible) in the EVA (40% VA) diluent and thus the weight percent concentration in the diluent corresponds approximately to the thermodynamic activity. Secoverine and dexsecoverine are solvents for the adhesive and form solutions therewith at concentrations of 300 mg/cm$^3$ or more. Adverse effects on adhesive properties have been observed when agent concentration reached about 50 mg/cm$^3$.

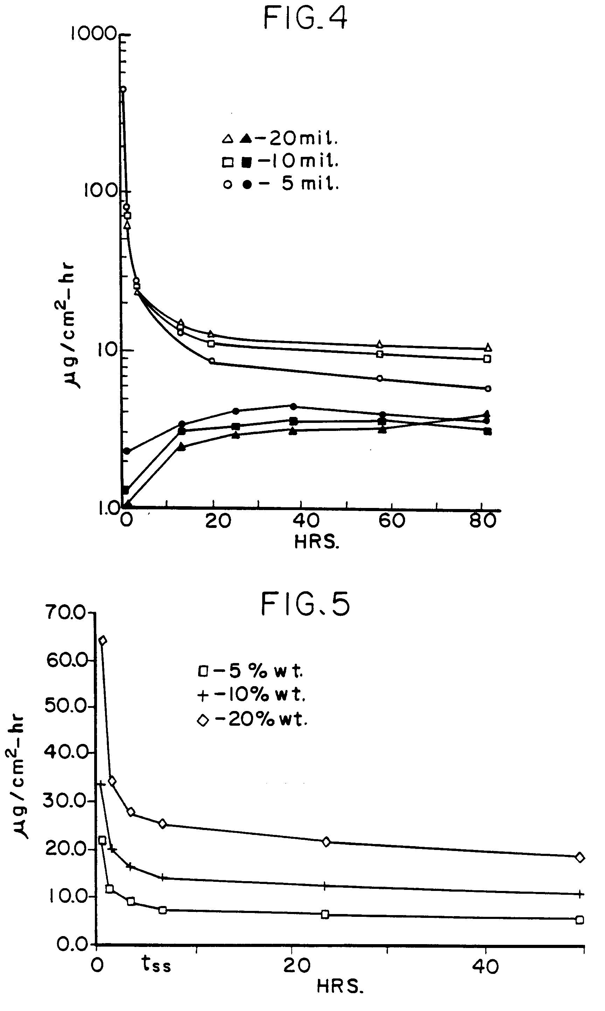

Thus, according to the preferred dexsecoverine delivering embodiments of this invention, it is desirable to maintain the agent concentration in the adhesive below about 45 mg/cm$^3$ which corresponds to an activity of about 0.15 in the drug reservoir and the adhesive layers. The thicknesses of the adhesive and rate controlling layers in the subsaturated system were selected to provide an initial pulse of about 225 ug/cm$^2$ to saturate the agent binding sites in the skin, the contribution to the pulse of each such layer being dependent on the thickness of the layer and the solubility of the agent in each layer. A thicker layer would provide a higher initial pulse and a thinner layer would provide a smaller initial pulse for the same initial activity. One or 1.3 mil LDPE and 2 or 4 mil EVA (9% VA) rate control membranes were utilized in the preferred embodiments and drug reservoirs of approximately 5-20 mils were tested. A 5 mil thickness was sufficient to prevent the activity of the agent in the reservoir 3 from decreasing by more than 30% during a four-day administration period. The *in vitro* release rates of various subsaturated dexsecoverine systems are compared to the characteristics for unit activity systems in Table II. In Figure 4 the upper group of curves shows the *in vitro* release rates at 32#C vs. time in hours directly into an aqueous sink and the lower group curves show the flux through cadaver skin at 32#C vs. time in hours into an aqueous sink from racemic secoverine systems and illustrate the effect of varying reservoir thicknesses on *in vitro* release rates and flux.

TABLE II

| Drug Source | Dexsecoverine | | | | | Secoverine | |
|---|---|---|---|---|---|---|---|
| Drug Activity | 1.00 | 0.06 | 0.15 | 0.10 | 0.20 | 0.20 | 0.20 |
| Membrane | LDPE | EVA (9%VA) | LDPE | EVA (9%VA) | LDPE | LDPE | LDPE |
| Membrane Thickness (mils) | 1.0 | 4.0 | 1.0 | 2.0 | 1.3 | 1.3 | 1.3 |
| Adhesive Thickness (mils) | 1.7 | 1.8 | 1.7 | 1.4 | 1.7 | 1.7 | 1.7 |
| Reservoir Thickness (mils) | 5 | 5.0 | 5.0 | 5.0 | 20.0 | 10.0 | 5.0 |
| Initial Burst (ug/cm$^2$): | | | | | | | |
| from membrane | 170 | 142 | 26 | 118 | | | |
| from adhesive | 1325 | 84 | 199 | 109 | | | |
| Total | 1495 | 226 | 225 | 227 | | | |
| Avg. Steady State *In vitro* Release Rate at 32#C (Icg/cm$^2$/hr) | 57 | 3.5 | 8.2 | 22 | | | |
| Range (over 24-96 hr) | 60-54 | 7.5-5.5 | 10-7 | 24-18 | | | |

We have determined that to achieve anti-spasmodic activity from the continuous transdermal administration of secoverine, approximately 1 to 10 nanograms/ml of dexsecoverine should be maintained in the plasma. We have also discovered that the permeability of average human skin when exposed to unit activity sources of either secoverine or dexsecoverine appears to be in the range of approximately 20 to 60 ug/cm$^2$/hr. In order to deliver adequate amounts of a drug from a reasonably sized system, a target steady-state *in vivo* delivery rate of dexsecoverine from 10-40 ug/hr was selected which rate can be readily achieved according to our invention in a rate controlled device of reasonable size of from about 5 to 60 cm$^2$. Delivery periods of about 3-5 days can be obtained from subsaturated devices of Table 2, and administration periods up to about 7 days can be attained by increasing the thickness of the reservoir to about 10 mils.

EXAMPLE IV

Subsaturated transdermal delivery devices similar to those of Example III, but intended to deliver benztropine base are fabricated having an agent reservoir diluent of EVA (40% VA), and a 1 mil LDPE rate-controlling membrane. Benztropine base is soluble to about 650 mg/g of EVA (40% VA). 2.5 cm$^2$ devices are fabricated using a highly permeable, amine resistant silicone adhesive available from Dow Corning, (X7-2920) or polyisobutylene/mineral oil adhesives, an impermeable backing, and an 8 mil-thick reservoir layer having an initial benzotropine loading of 5, 10, and 20 weight percent equivalent to activities of 0.125, 0.25, and 0.5. The approximate *in vitro* release rates directly into an aqueous bath at 32-35#C to be obtained from such devices, using 1 mil LDPE rate-controlling membranes, are illustrated in Figure 5. The effect of using a 2-mil LDPE rate-controlling membrane is illustrated in Figure 6.

The permeability of average skin to benztropine is in the range of 70 to 90 ug/cm$^2$ hr and systems as described above can deliver benztropine *in vivo* at therapeutically useful rates of 10 to 40 ug/hr. The size of the device can be selected to provide daily doses of about 0.4 to 4.5 mg for up to 4 days.

EXAMPLE V

Benztropine transdermal delivery devices for use in clinical testing were fabricated as set forth generally in Example IV from a 10% benztropine in 90% EVA 40 reservoir composition into 5 cm$^2$ patches using 1.5 mil LDPE rate controlling membranes and 1.8 mil amine resistant adhesive layers. With a 5 mil reservoir layer the devices contained about 6.4 mg of benztropine and are intended for a 24 hour administration period. The *in vitro* release rate vs. time at 32#C into an aqueous sink is shown in Figure 7. When applied to human subjects on a daily basis, anticholinergically effective transdermal delivery of benztropine can be obtained.

Having thus generally described our invention and preferred embodiments thereof, it is apparent that various modifications and substitutions will be apparent to workers skilled in the art, which can be made without departing from the scope of our invention which is limited only by the following claims wherein:

**Claims**

1. A transdermal delivery device (1) for delivering an agent over a predetermined administration period comprising a reservoir (3) containing an agent and a diluent, release rate controlling means through which said agent, but not said diluent, permeates in use from the device to the skin of a patient, and in-line adhesive means through which said agent must pass to reach the skin (5), wherein said agent is a solvent for the adhesive, characterised in that the initial equilibrated activity of said agent in said reservoir is below saturation and at a level at which the adhesive means (5) retains adhesive properties, and the initial loading of the agent in the reservoir is sufficient to prevent the activity of the agent in the reservoir from decreasing by more than 75% during the predetermined administration period.

2. The device of claim 1 wherein at least 50% of the initial equilibrated loading of the agent in the device is in the reservoir (2).

3. The device of Claim 1 or Claim 2 wherein at least 75% of the initial equilibrated loading of the agent in the device is in the reservoir (3).

4. The device of claim 1, Claim 2 or claim 3, wherein the device has a predetermined administration period of up to 7 days, and the initial loading of the agent is at a level that the decrease in activity during said predetermined administration period is no greater than 75%.

5. The device of claim 4 wherein said decrease in activity is no greater than 25%.

6. The device of Claim 4 or Claim 5 wherein said predetermined administration period is from 8 hours to 3 days.

7. The device of any preceding claim wherein said agent is an oily, non-polar material, liquid at body temperature.

8. The device of any preceding claim wherein said agent is selected from ethanol, polyethylene glycol monolaurate, glycerol monolaurate, glycerol monooleate, nicotine, arecoline, secoverine, dexsecoverine and benztropine.

9. The device of any preceding claim wherein said agent is selected from nicotine, dexsecoverine and benztropine.

10. The device of any preceding claim wherein the diluent for said agent is an ethylene vinyl acetate copolymer.

11. The device of any preceding claim wherein said agent release rate controlling means is an agent release rate controlling membrane (4).

12. The device of Claim 11 wherein said release rate controlling membrane is of low density polyethylene or ethylene vinyl acetate copolymer.

13. The device of any of any preceding claims wherein said agent is nicotine, being contained in an amount sufficient to administer nicotine through human skin at a flux rate from 35 to 200 $\mu$g/cm$^2$/h for at least 24 hours.

14. The device of claim 13 wherein the administration rate is from 250 to 4000 $\mu$g/h.

15. The device of any preceding claim wherein said reservoir comprises from 5 to 50 wt.% nicotine and from 50 to 95 wt.% ethylene vinyl acetate copolymer having a vinyl acetate content in the range of 28 to 60%.

16. The device of any of claims 1-12 wherein the agent is nicotine.

17. The device of any preceding claim wherein the initial equilibrated activity of the agent in the reservoir does not exceed 0.50.

18. A device as claimed in claim 16 or claim 17 wherein said initial equilibrated activity is in the range of 0.05-0.50.

19. A device as claimed in Claim 16 or Claim 17 characterised by rate controlling means for controlling diffusion of nicotine from a skin-facing side of the reservoir initially at a flux of about 1017 $\mu$g/cm$^2$hr decreasing to a target steady state in vivo delivery rate within the range of 5 - 800 $\mu$g/cm$^2$hr for a substantial portion of the predetermined administration period.

20. A device as claimed in Claim 16 or Claim 17 characterised by an initial nicotine loading and nicotine permeable rate controlling means for controlling the diffusion of nicotine from the skin facing side at a flux between 23.1$\mu$g/cm$^2$hr and 133.2$\mu$g/cm$^2$hr during the period between 2 and 30.75 hours after beginning delivery from the device in service.

21. A method for administering nicotine which comprises:

(a) applying a device as claimed in any preceding claim to the skin of a patient when the thermodynamic activity of said nicotine in said reservoir is no greater than 0.50; and

(b) maintaining the concentration of nicotine in said reservoir throughout a predetermined administration period at a level sufficient to prevent the activity from decreasing by more than 75%.

22. A method as claimed in claim 23 wherein the activity when applied is in the range of 0.05-0.50.

23. A method for administering nicotine which comprises:

(a) applying a device as claimed in any of claims 13-22 to the skin of the patient; and

(b) removing it at the end of said predetermined administration period.

24. The method of claim 23 wherein said administration period is 24 hours.

25. The method of claim 23 wherein said administration period is 16 hours.

**Patentansprüche**

1. Transdermale Abgabevorrichtung zur Abgabe eines Wirkstoffes über einen vorgegebenen Verabreichungszeitraum, umfassend ein den Wirkstoff und ein Verdünnungsmittel enthaltendes Reservoir (3), eine zur Steuerung der Abgaberate bestimmte Einrichtung , durch die hindurch der Wirkstoff, nicht jedoch das Verdünnungsmittel im Gebrauch von der Vorrichtung zur Haut des Patienten übertritt, und im Weg des Wirkstoffs liegende Klebemittel, durch die der Wirkstoff hindurchtreten muß, um die Haut zu erreichen, wobei der Wirkstoff ein Lösungsmittel für den Klebstoff ist, dadurch **gekennzeichnet**, daß die anfängliche Gleichgewichtsaktivität des Wirkstoffes in dem Reservoir unter dem Sättigungswert und auf einem Niveau liegt, bei dem die Klebemittel ihre Hafteigenschaft behalten, und daß der anfängliche Wirkstoffgehalt im Reservoir ausreicht, um während des vorgegebenen Verabreichungszeitraumes ein Absinken der Aktivität des Wirkstoffes in dem Reservoir um mehr als 75 % zu verhindern.

2. Vorrichtung nach Anspruch 1, dadurch **gekennzeichnet**, daß mindestens 50 % des anfänglichen Gleichgewichtsgehaltes an Wirkstoff in der Vorrichtung sich im Reservoir (3) befindet.

3. Vorrichtung nach Anspruch 1 oder 2, dadurch **gekennzeichnet**, daß mindestens 75 % des anfänglichen Gleichgewichtsgehaltes des Wirkstoffes in der Vorrichtung sich im Reservoir (3) befindet.

4. Vorrichtung nach Anspruch 1, 2 oder 3, dadurch **gekennzeichnet**, daß sie einen vorgegebenen Verabreichungszeitraum von bis zu 7 Tagen hat und daß der anfängliche Wirkstoffgehalt auf einem solchen Niveau liegt, daß die Abnahme der Aktivität während des vorgegebenen Verabreichungszeitraumes nicht größer als 75 % ist.

5. Vorrichtung nach Anspruch 4, dadurch **gekennzeichnet**, daß die Abnahme der Aktivität nicht größer als 25 % ist.

6. Vorrichtung nach Anspruch 4 oder 5, dadurch **gekennzeichnet**, daß der vorgegebene Verabreichungszeitraum von 8 Stunden bis zu 3 Tagen reicht.

7. Vorrichtung nach einem der vorangegangenen Ansprüche, dadurch **gekennzeichnet**, daß der Wirkstoff ein öliges nicht polares Material ist, das bei Körpertemperatur flüssig ist.

8. Vorrichtung nach einem der vorangegangenen Ansprüche, dadurch **gekennzeichnet**, daß der Wirkstoff aus der Ethanol, Polyethylenglykolmonolaurat, Glycerolmonolaurat, Glycerinmonooleat, Nikotin, Arecolin, Sekoverin, Dexsekoverin und Benztropin umfassenden Stoffgruppe gewählt wird.

9. Vorrichtung nach einem der vorangegangenen Ansprüche, dadurch **gekennzeichnet**, daß der Wirkstoff aus der Nikotin, Dexsekoverin und Benztropin umfassenden Stoffgruppe gewählt ist.

10. Vorrichtung nach einem der vorangegangenen Ansprüche, dadurch **gekennzeichnet**, daß das Verdünnungsmittel für den Wirkstoff ein Ethylenvinylacetat-Copolymer ist.

11. Vorrichtung nach einem der vorangegangenen Ansprüche, dadurch **gekennzeichnet**, daß die Einrichtung zur Steuerung der Wirkstoffreigaberate eine Membran (4) zur Steuerung der Wirkstoffreigaberate ist.

12. Vorrichtung nach Anspruch 11, dadurch **gekennzeichnet**, daß die Membran zur Steuerung der Freigaberate ein Polyethylen oder Ethylenvinylacetat-Copolymer geringer Dichte ist.

13. Vorrichtung nach einem der vorangegangenen Ansprüche, dadurch **gekennzeichnet**, daß der Wirkstoff Nikotin ist, das in einer ausreichenden Mengen vorhanden ist, um Nikotin durch die menschliche Haut mit einer Durchtrittsgeschwindigkeit von 35 bis 200 $\mu g/cm^2/h$ für mindestens 24 Stunden zu verabreichen.

14. Vorrichtung nach Anspruch 13, dadurch **gekennzeichnet**, daß die Verabreichungsgeschwindigkeit 250 bis 4000 $\mu g/h$ beträgt.

15. Vorrichtung nach einem der vorangegangenen Ansprüche, dadurch **gekennzeichnet**, daß das Reservoir zwischen 5 und 50 Gewichtsprozenten Nikotin und zwischen 50 bis 95 Gewichtsprozenten Ethylenvinylacetat-Copolymer enthält, das einen Vinylacetat-Gehalt von ca. 28 bis 60 % hat.

16. Vorrichtung nach einem der Ansprüche 1 bis 12, dadurch **gekennzeichnet**, daß der Wirkstoff Nikotin ist.

**17.** Vorrichtung nach einem der vorangegangenen Ansprüche, dadurch **gekennzeichnet**, daß die anfängliche Gleichgewichtsaktivität des Wirkstoffes in dem Reservoir 0,50 nicht überschreitet.

**18.** Vorrichtung nach Anspruch 16 oder 17, dadurch **gekennzeichnet**, daß die anfängliche Gleichgewichtsaktivität im Bereich von 0,05 bis 0,5 liegt.

**19.** Vorrichtung nach Anspruch 16 oder 17, **gekennzeichnet** durch eine Geschwindigkeitssteuereinrichtung zum Steuern der Diffusion von Nikotin aus einer der Haut zugewandten Seite des Reservoirs mit einer anfänglichen Flußgeschwindigkeit von ca. 1017 $\mu$g/cm$^2$/h, die auf eine in-vivo-Gleichgewichtsabgabegeschwindigkeit im Gleichgewichtszustand abnimmt, die zwischen 5 bis 800 $\mu$g/cm$^2$/h für einen wesentlichen Abschnitt des vorgegebenen Verabreichungszeitraumes beträgt.

**20.** Vorrichtung nach Anspruch 16 oder 17, **gekennzeichnet** durch einen anfänglichen Nikotingehalt und eine Nikotinwanderungsgeschwindigkeitssteuereinrichtung zum Steuern der Diffusion von Nikotin aus der der Haut zugewandten Seite mit einer Durchflußrate zwischen 23,1 $\mu$g/cm$^2$/h und 133,2 $\mu$g/cm$^2$/h während des Zeitraumes zwischen 2 und 30,75 Stunden nach dem Beginn der Abgabe aus der im Gebrauch befindlichen Vorrichtung.

**21.** Verfahren zum Verabreichen von Nikotin, umfassend:

(a) Anbringen einer Vorrichtung nach einem der vorangegangenen Ansprüche auf der Haut eines Patienten, wenn die thermodynamische Aktivität des Nikotins in dem Reservoir nicht größer als 0,5 ist; und

(b) Aufrechterhalten der Konzentration des Nikotins in dem Reservoir während einer vorgegebenen Verabreichungsperiode auf einem Niveau, das ausreicht, um ein Absinken der Aktivität um mehr als 75 % zu verhindern.

**22.** Verfahren nach Anspruch 21, dadurch **gekennzeichnet**, daß die Aktivität im Anwendungsfall in dem Bereich von 0,05 bis 0,5 liegt.

**23.** Verfahren zum Verabreichen von Nikotin, umfassend:

(a) Ansetzen einer Vorrichtung nach einem der vorangegangenen Ansprüche 13 bis 20 an die Haut des Patienten und

(b) Entfernen der Vorrichtung am Ende des vorgegebenen Verabreichungszeitraumes.

**24.** Verfahren nach Anspruch 23, dadurch **gekennzeichnet**, daß der Verabreichungszeitraum 24 Stunden beträgt.

**25.** Verfahren nach Anspruch 23, dadurch **gekennzeichnet**, daß der Verabreichungszeitraum 16 Stunden beträgt.

**Revendications**

**1.** Un dispositif de distribution transcutanée (1) pour délivrer un agent sur une période d'administration prédéterminée comprenant un réservoir (3) contenant un agent et un diluant, un moyen de commande du taux de libération au travers duquel ledit agent mais non ledit diluant passe à travers lors de l'utilisation du dispositif à la peau d'un patient et un moyen adhésif en ligne au travers duquel ledit agent doit passer pour atteindre la peau (5), dans lequel ledit agent est un solvant pour l'adhésif, caractérisé en ce que l'activité équilibrée initiale dudit agent dans ledit réservoir est en dessous de la saturation et à un niveau auquel le moyen adhésif (5) conserve les propriétés adhésives et la charge initiale de l'agent dans le réservoir est suffisante pour empêcher l'activité de l'agent dans le réservoir de diminuer de plus de 75% pendant la période d'administration prédéterminée.

**2.** Le dispositif selon la revendication 1, dans lequel au moins 50% de la charge équilibrée initiale de l'agent dans le dispositif est dans le réservoir (2).

**3.** Le dispositif selon la revendication 1 ou la revendication 2, dans lequel au moins 75% de la charge équilibrée initiale de l'agent dans le dispositif est dans le réservoir (3).

**4.** Le dispositif selon la revendication 1, la revendication 2 ou la revendication 3, dans lequel le dispositif a une période d'administration prédéterminée allant jusqu'à 7 jours et la charge initiale de l'agent est à un niveau tel que la diminution d'activité pendant ladite période d'administration prédéterminée n'est pas supérieure à 75%.

5. Le dispositif selon la revendication 4, dans lequel ladite diminution dans l'activité n'est pas supérieure à 25%.

6. Le dispositif selon la revendication 4 ou la revendication 5, dans lequel ladite période d'administration prédéterminée est de 8 heures à 3 jours.

7. Le dispositif selon l'une quelconque des revendications précédentes, dans lequel ledit agent est un liquide huileux, matériau non-polaire à la température du corps.

8. Le dispositif selon l'une quelconque des revendications précédentes, dans lequel ledit agent est choisi parmi l'éthanol, le monolaurate de polyéthylène glycol, le monolaurate de glycérol, le monooléate de glycérol, la nicotine, l'arécoline, la sécovérine, la dexsécovérine et la benzotropine.

9. Le dispositif selon l'une quelconque des revendications précédentes, dans lequel ledit agent est choisi parmi la nicotine, la dexsécovérine et la benzotropine.

10. Le dispositif selon l'une quelconque des revendications précédentes, dans lequel le diluant pour ledit agent est un copolymère éthylène-acétate de vinyle.

11. Le dispositif selon l'une quelconque des revendications précédentes, dans lequel ledit moyen de commande du taux de libération de l'agent est une membrane de commande du taux de libération de l'agent (4).

12. Le dispositif selon la revendication 11, dans lequel ladite membrane de commande du taux de libération est du polyéthylène basse densité ou un copolymère éthylène-acétate de vinyle.

13. Le dispositif selon l'une quelconque des revendications précédentes, dans lequel ledit agent est la nicotine, contenue selon une quantité suffisante pour administrer de la nicotine au travers de la peau humaine à un débit de 35 à 200 $\mu$g/cm2/h pendant au moins 24 heures.

14. Le dispositif selon la revendication 13, dans lequel le taux d'administration est de 250 à 4000 $\mu$g/h.

15. Le dispositif selon l'une quelconque des revendications précédentes, dans lequel ledit réservoir comprend de 5 à 50% en poids de nicotine et de 50 à 95% en poids de copolymère éthylène-acétate de vinyle ayant une teneur en acétate de vinyle dans la gamme de 28 à 60%.

16. Le dispositif selon l'une des revendications 1 à 12 dans lequel l'agent est la nicotine.

17. Le dispositif selon l'une quelconque des revendications précédentes dans lequel l'activité équilibrée initiale de l'agent dans le réservoir ne dépasse pas 0,50.

18. Un dispositif selon la revendication 16 ou la revendication 17, dans lequel ladite activité équilibrée initiale se situe dans la gamme de 0,05-0,50.

19. Un dispositif selon la revendication 16 ou la revendication 17, caractérisé en ce que le moyen de commande du taux pour commander la diffusion de la nicotine à partir d'un côté tourné vers la peau du réservoir est initialement à un débit d'environ 1017 $\mu$g/cm2/h diminuant jusqu'à un taux de distribution in vivo à l'état constant cible dans la gamme de 5 à 800 $\mu$g/cm2/h pour une partie sensible de la période d'administration prédéterminée.

20. Un dispositif selon la revendication 16 ou la revendication 17, caractérisé par une charge de nicotine initiale et un moyen de commande du taux perméable de nicotine pour commander la diffusion de nicotine à partir du côté tourné vers la peau à un débit entre 23,1 $\mu$g/cm2/h et 133,2 $\mu$g/cm2/h pendant la période entre 2 et 30,75 heures après le début de la distribution à partir du dispositif en service.

21. Un procédé pour administrer de la nicotine qui consiste:

(a) à appliquer un dispositif selon l'une quelconque des revendications précédentes à la peau d'un patient lorsque l'activité thermodynamique de ladite nicotine dans ledit réservoir n'est pas supérieure à 0,50; et

(b) à maintenir la concentration de la nicotine dans ledit réservoir pendant une période d'administration prédéterminée à un niveau suffisant pour empêcher l'activité de diminuer de plus de 75%.

22. Un procédé selon la revendication 23, dans lequel l'activité lors de l'application se situe dans la gamme de 0,05 à 0,50.

23. Un procédé pour administrer de la nicotine qui consiste:

(a) à appliquer un dispositif selon l'une quelconque des revendications 13 à 22 à la peau du patient; et
(b) à le retirer à la fin de ladite période d'administration prédéterminée.

24. Le procédé selon la revendication 23 dans lequel ladite période d'administration est de 24 heures.

25. Le procédé selon la revendication 23 dans lequel ladite période d'administration est de 16 heures.

FIG.1

FIG.2

FIG.3

# FIG.4

△▲—20 mil.
□■—10 mil.
○●— 5 mil.

μg/cm²-hr

HRS.

# FIG.5

□—5 % wt.
+—10% wt.
◇—20% wt.

μg/cm²-hr

t_ss

HRS.

## FIG.6

## FIG. 7